# EUROPEAN PATENT SPECIFICATION

(11) **EP 1 922 025 B1**
(45) Date of publication and mention of the grant of the patent: **23.03.2016**
(21) Application number: 06814208.2
(22) Date of filing: 05.09.2006
(51) Int. Cl.: A61L 31/16, A61F 2/07, A61F 2/89, A61F 2/90, C08L 27/18, A61L 31/04, A61F 2/06

(54) **DRUG-RELEASING GRAFT**
ARZEINMITTELFREISETZENDES IMPLANTAT
GREFFON DE DIFFUSION DE MEDICAMENTS

(30) Priority: 06.09.2005 US 714724 P
(43) Date of publication of application: 21.05.2008
(73) Proprietor: C.R.Bard, Inc., Murray Hill, NJ 07974 (US)
(72) Inventor: MCDERMOTT, John, D., Chandler, AZ 85248 (US); CASANOVA, Robert, Michael, Scottsdale, AZ 85259 (US); PATHAK, Chandrashekhar, P, Phoenix, Arizona 85044 (US)
(74) Representative: Hoffmann Eitle
(86) International application number: PCT/US2006/034671
(87) International publication number: WO 2007/030512

(56) References cited:
- EP-A2- 0 734 721
- WO-A1-02/055121
- WO-A1-02/055122
- US-A- 5 102 402
- US-A- 5 605 696
- US-A- 5 893 840
- US-A1- 2004 117 006
- US-A1- 2004 122 509
- US-A1- 2005 004 661

## Description

### BACKGROUND OF THE INVENTION

Synthetic grafts are routinely used to restore the blood flow in patients suffering from vascular diseases. For example, prosthetic grafts made from expanded polytetrafluoroethylene (ePTFE) are commonly used and have shown favorable patency rates, meaning that depending on a given time period, the graft maintains an open lumen for the flow of blood therethrough. Patency rates may vary depending on the implantation site, graft design, graft surface chemistry, surface morphology, texture, porosity and graft diameter.

Various mechanical, biological and/or chemical conditions can result in failure of synthetic polymeric grafts. Possible cause of synthetic polymeric graft failure may include thrombosis and intimal hyperplasia at or near the graft anastamotic site. Thrombosis may be controlled by taking oral anticoagulation therapies or graft surface modification chemistries. Intimal hyperplasia on the other hand can be difficult to control. Intimal hyperplasia may be caused by proliferation and migration of smooth muscle cells from the media to the intimal of the graft. The growth and proliferation of smooth muscle cells produces extracellular matrix materials which can cause disruption and blockage of blood flow. Other tissue growth and deposition of substances on the synthetic grafts may also result in blockage of the synthetic grafts.

Therefore, methods for incorporating drugs onto a medical device may be particularly useful in vascular graft implantation. A drug integrated into a vascular graft can help maintain patency of the vascular graft after implantation.
US 2004/0122509 A1 relates to a radiopaque medical prosthesis in the form of an ePTFE vascular graft. In the porous structure of the ePTFE material, therapeutic agents can be held, which may be added to the prosthesis by addition of a therapeutic drug solution under pressure.
WO 02/055122 A1, WO 02/055121 A1, and EP 0 734 721 A2 are further prior art documents.

### SUMMARY OF THE INVENTION

Localized delivery of drugs from an implanted medical device can be utilized to provide targeted therapeutics in a specific region of the patient's body. For example, drug(s) embedded in a vascular graft can be used to prevent occlusion and minimize partial impairment of the blood flow in the implanted device. Various drugs, including but not limited to anti-inflammatory substance, anti-coagulant substance, agents that suppresses cellular growth, etc., can be incorporated into the structure of an implantable medical device and then released into the surrounding tissue once the medical device has been implanted.

In one variation of incorporating a drug into a medical device, a water insoluble drug is incorporated into the structure of an implantable medical device as solid crystals using the method of claim 1. The implantable device embedded with the solid crystals can then be deployed in a patient's body. Post implantation, the crystal in the implantable device will slowly dissolve and diffuse into the tissue surrounding the implantable device. In one implementation, at least a portion of the implantable device includes a porous structure, in which drug crystals can be incorporated therein. In another variation, the implantable device includes both a polymeric material and a metal alloy. The metal alloy may be configured with a lattice or scaffold to provide the underlying structure support for the implantable device.

One potential method for incorporating a drug into a medical device includes first dissolving a water insoluble drug in an organic solvent and then immersing the implantable medical device in the organic solvent. Once the organic solvent with the drug has permeated at least portion of the medical device, the implantable device is removed from the organic solvent. The organic solvent is allowed to evaporate, resulting in drug crystals forming within and/or over the structure of the implantable device. Heating, air flow, vacuum and other methods well known to one skilled in the art may be implemented to facilitate the removal of the organic solvent and/or formation of the drug crystals.

In one example, a water insoluble drug is dissolved in an organic solvent, such as ethanol. An ePTFE graft is then immersed within this organic solvent. Once the organic solvent carrying the dissolved drug has permeated into at least a portion of the porous ePTFE structure, the ePTFE graft is extracted from the solvent. The organic solvent is removed from the ePTFE graft leaving the drug, which form crystals in the porous structure within the ePTFE graft. The resulting ePTFE graft with embedded drug crystals can then be implanted into a patient's body.

These and other embodiments, features and advantages of the present invention will become more apparent to those skilled in the art when taken with reference to the following more detailed description of the invention in conjunction with the accompanying drawings that are first briefly described herein.

### BRIEF DESCRIPTION OF THE DRAWINGS

FIG. 1 is a diagram illustrating one variation of a method for incorporating a drug into an implantable medical device. A drug is loaded within the structure of the implantable medical device as crystals and then released into the patient's body post implantation.
FIG. 2 shows an example of a vascular graft fabricated from a porous ePTFE tubing. The method described in FIG. 1 can be utilized to form drug crystals within the porous ePTFE tubing.
FIG. 3 shows another example of a vascular graft fabricated using porous polymer. In this particular example, the vascular graft is designed for bypass applications.
FIG. 4 shows an example of a porous polymer based vascular graft having a bifurcation.
FIG. 5A is an SEM picture of an ePTFE graft node-fibril microstructure.
FIG. 5B is an SEM picture of an ePTFE graft node-fibril microstructure with drug crystals incorporated therein.
FIG. 6 illustrates one example where the vascular graft is configured with a porous polymeric tubing embedded with flexible metal alloy lattice to provide structural support.
FIG. 7 shows a stent graft fabricated from a thin and flexible ePTFE layer placed over a collapsible nitinol lattice.

### DETAILED DESCRIPTION OF THE PREFERRED EMBODIMENTS

The following detailed description should be read with reference to the drawings, in which identical reference numbers refer to like elements throughout the different figures. The drawings, which are not necessarily to scale, depict selective embodiments and are not intended to limit the scope of the invention. The detailed description illustrates by way of example, not by way of limitation, the principles of the invention. This description would enable one skilled in the art to make and use the invention, and describes several embodiments, adaptations, variations, alternatives and uses of the invention, including what is presently believed to be the best mode of carrying out the invention.

Before describing preferred embodiments, it is to be understood that unless otherwise indicated, this invention need not be limited to applications in humans. As one skilled in the art would appreciate, variations of the invention may be applied to other mammals as well. Moreover, it should be understood that embodiments of the present invention may be applied in combination with various catheters, introducers or other implantation and connection devices for placement of the implantable device into a patient's body.

Synthetic vascular grafts and stents are used herein as examples of the types of implantable devices that can be loaded with crystallized drug for delivery into a patient's body, in order to illustrate the various aspects of the invention disclosed herein. In light of the disclosure herein, one skilled in the art would appreciate that the drug loading methods described herein may be utilized for incorporation of various suitable drugs into implantable devices for localized drug delivery. One skilled in the art having the benefit of this disclosure would also appreciate that drugs that are suitable for implementation with methods described herein includes, but not limited to, various pharmaceutical agents, antithrombogenic agents, anti-inflammatory agents, antibacterial agents, anti-viral agents, etc.

It must also be noted that, as used in this specification and the appended claims, the singular forms "a," "an" and "the" include plural referents unless the context clearly dictates otherwise. Thus, for example, the term "a branch" is intended to mean a single branch or a combination of branches, "a polymer" is intended to mean one or more polymers, or a mixture thereof. In addition, water insoluble drug as used herein includes drugs that are sparingly soluble in water. Examples of water insoluble drugs include, but not limited to, paclitaxel, lovastatin, simvastatin, chlorhexindene acetate, rapamycin, and combinations thereof. Preferably the drug has a solubility range of about 0.001% to about 5%. More preferably, the drug has a solubility range of about 0.1 % to about 3%, and most preferably about 0.1% to about 2%. As used herein, the term "about" and "approximately" for any numerical values indicate a suitable range of tolerance that would permit an embodiment to function for its intended purpose as a drug releasing prosthesis.

Disclosed herein are implantable medical devices embedded with drug crystals for local drug delivery and method of incorporation the drug crystals within the underlying structure of the implantable medical device. In one variation, a drug configured to suppress cellular growth and/or prevent thrombus formation is incorporated into a porous polymeric structure on a medical device. The medical device can be adapted for implantation within the circulatory system of a patient. For example, a vascular graft with at least a portion of its structure formed of a porous polymeric material can serve as underlying matrix for receiving the drug solution and allowing the formation of drug crystal therein.

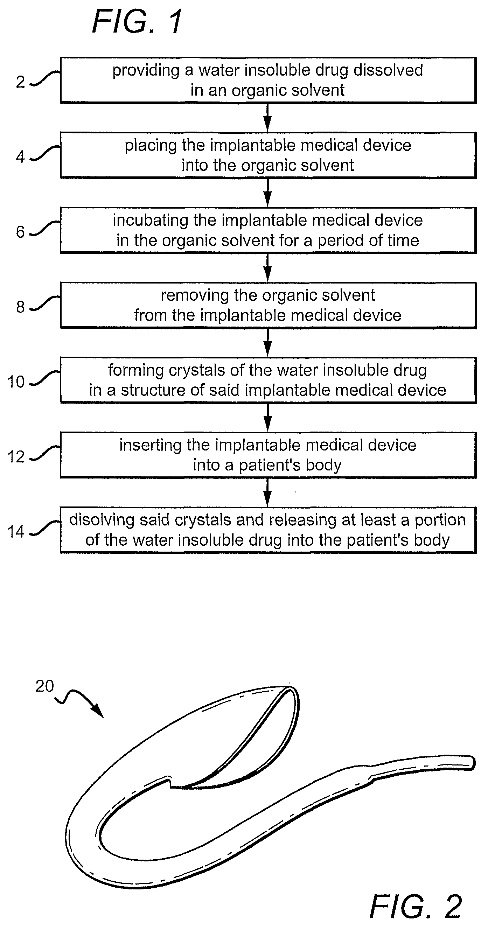

In another variation, the method of loading a drug into an implantable medical device includes first dissolving a water insoluble drug into an organic solvent. The organic solvent with the water insoluble drug is then provided as the medium to deliver the drug into the structure of the implantable medical device 2, as illustrated in FIG. 1. The implantable medical device is then immersed into the organic solvent 4 and incubated in the organic solvent for a period of time 6 (e.g., 1 second, 5 minutes, 10 minutes, 24 hours, etc.). A pressure gradient, fluid flow, or agitation, may be generated across and/or around the immersed medical device to facilitate the permeation of the medical device with the organic solvent. After the organic solvent has permeated at least portion of the implantable medical device, it is extracted from the organic solvent. The residual organic solvent on the medical device is then removed, for example, by evaporation 8. As the organic solvent is removed from the medical device, crystals of the water insoluble drug are formed within the structure of the implantable medical device 10. The implantable medical device embedded with the drug crystals can then be deployed in a patient's body 12. Once the implantable medical device is placed in a patient's body, the drug crystal will dissolve over time and release the drug into the tissue surrounding the implantable medical device 14. In one variation, the bodily fluid (e.g., blood, etc.) and/or the corresponding warm temperature (e.g., about 37 degree Celsius) causes the crystal to dissolve and release the drug.

In one example, at least a portion of the structure forming the implantable medical device includes a porous polymer (e.g., ePTFE, etc.), and crystals are formed inside the pores of the porous polymer. In another example, a generally tubular shaped polymeric structure is utilized to manufacture a vascular graft for implantation in a patient's vascular system. In yet another example, the tubular shaped polymeric structure is configured as a stent. A lattice includes of a metal alloy (e.g. Nitinol, etc.) can be integrated within the porous polymeric tubular structure to form a collapsible framework.

In another implementation, a vascular graft fabricated from expanded polytetrafluoroethylene (ePTFE) is utilized to receive the drug. The expanded ePTFE graft has a porous microstructure of nodes interconnected by fibrils, the microstructure being typically characterized by internodal distance (IND), or the distance between nodes at a given location of the microstructure (e.g., inner surface, outer surface, average throughout wall thickness, etc.). In one variation, ePTFE grafts with an IND in the range of about 10 microns to about 70 microns are utilized to receive the drug. Depending on the IND of the graft at the outer surface thereof, the porous microstructure may permit tissue in-growth to anchor the graft at the implanted site. Crystallized drug is formed in the porous microstructure of the ePTFE graft, such that once the graft is implanted in a patient, the drug crystals dissolve over time, releasing drug into the surrounding tissue.

In one variation, a water insoluble drug (e.g., paclitaxel, lovastatin, simvastatin, chlorhexidine acetate, rapamycin, etc.) is incorporated into the porous polymeric microstructure. For example, a bioactive drug that is sparingly soluble in water is used to form crystals within the porous microstructure of an ePTFE graft. The drug crystal-ePTFE graft is then implanted into a patient's body as a vascular conduit/graft. Upon implantation, the drug crystals dissolve slowly in the surrounding tissue. The solubilized drug creates a local therapeutic effect. For example, the selective drugs may be implemented to reduce smooth muscle proliferation in and around the vascular graft, and thus, prevent occlusion of the vascular graft.

In one exemplary approach, paclitaxel, an anticancer drug and a cell cycle inhibitor is dissolved in organic solvent such as ethanol to make a 10% solution. As one of skilled in the art having the benefit of this disclosure would appreciate, various other organic solvents can also be used to dissolve paclitaxel. Organic solvents that can be used include, but are not limited to, tetrahydrofuran, ethanol, isopropanol, acetone, ethyl acetate, hexane, octane, and the like. In one variation solvents with low toxicity and low boiling point are utilized.

An ePTFE vascular graft (e.g., 6 mm diameter, 20 cm in length, C. R. Bard catalogue number V2006C, or similar graft with or without carbon lining) is placed in the paclitaxel solution prepared in ethanol. In one variation, an ePTFE graft impregnated with carbon is implemented to receive the drug. The vascular graft is then incubated at ambient temperature for a period of time (e.g., 10 minutes, etc.). After incubation, the vascular graft is removed from the solution and allowed to dry. In one variation, the graft is hung in the air at room temperature to allow the solvent, ethanol, to evaporate. During the removal/evaporation of ethanol, the paclitaxel crystals are formed within the porous structure of the graft. The presence of paclitaxel crystals may be verified by observation under a microscope. Other methods may also be implemented to facilitate the removal of the solvent. For example, in one variation the graft can be placed in a vacuum chamber. In another variation, a heated lamp or other heating device may be utilized to heat the graft. Airflow can also be generated over the graft to facilitate the evaporation process.

Once the vascular graft is incorporated with paclitaxel crystals, it is implanted in a patient's body. Post implantation, the paclitaxel crystals will dissolve over time into the surrounding tissue. The dissolved paclitaxel can produce a local therapeutic effect. For example, the dissolved paclitaxel may suppress the growth of smooth muscle cell within the lumen of the vascular graft. Because paclitaxel is relatively insoluble in saline solution, once implanted inside a patient's body, it will dissolve slowly over an extended period of time (e.g., 3 to 180 days).

In one variation, the manufacturer can fabricate grafts with varying concentration of paclitaxel by controlling the amount of paclitaxel crystals that are formed within the graft. For example, one can modify the concentration of the drug in the organic solution in order to control the amount of drug crystals formed in the porous polymeric structure of the implantable medical device. In one application, ethanol solutions with varying paclitaxel concentration (e.g., in various ranges between about 0.1% to about 30%) can be utilized to prepare vascular grafts with varying amounts of paclitaxel. The above drug loading technique permits one to integrate water insoluble or sparingly water soluble drugs into a medical device without the need to first combine the drug with an intermediate polymeric-carrier. The use of the polymeric-carrier to bind the drug, and then insert the polymeric-carrier into the medical device, can be a tedious process. Furthermore, the process of binding the drug to the polymeric-carrier may denature or otherwise damage the bioactivity of the drug. In addition, the non-use of polymeric-carrier to insert the drug into the vascular graft simplifies graft design and permits the use of ePTFE surface for blood and tissue contact.

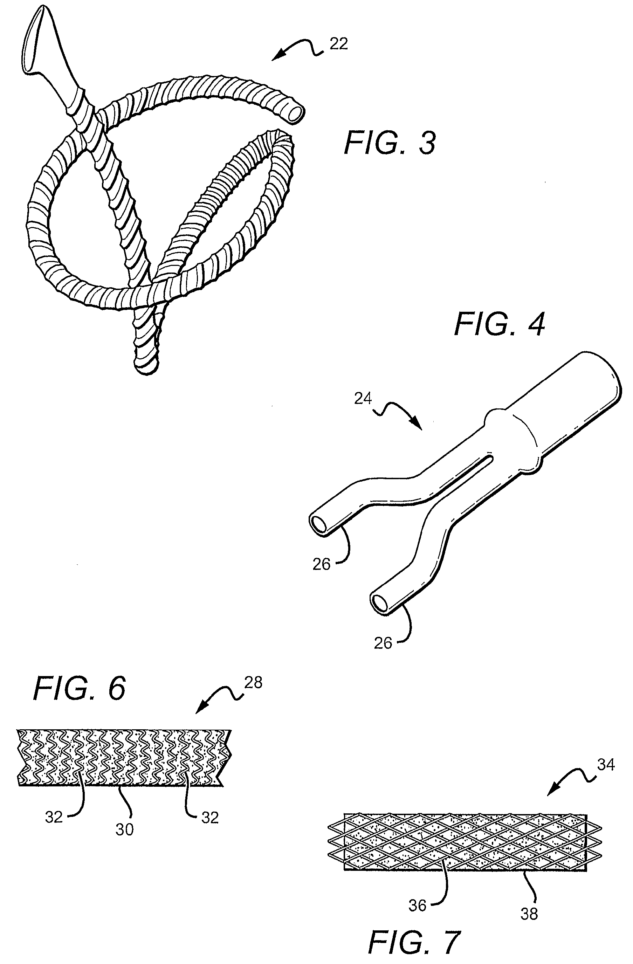

One skilled in the art having the benefit of this disclosure would appreciate that the method described herein can be utilized to incorporate drug crystals into to various medical device having porous structures. FIG. 2 shows an example of an ePTFE vascular graft 20 (Venaflo™, C. R. Bard, Murray Hill, NJ) configured for hemodialysis applications. The ePTFE vascular graft is immersed in an organic solvent with paclitaxel, and incubated for 10 minutes. The ePTFE graft is then removed from the organic solvent and air dried. As the organic solvent in the vascular graft evaporates, crystals of paclitaxel are formed within the pores of the ePTFE polymer on the vascular graft. FIG. 3 and 4 illustrate additional variations of vascular graft fabricated from porous polymers that are biocompatible, such that drug crystals can be loaded into the polymer according to the method described above. FIG. 3 shows an example of bypass graft 22 (Dynaflo™ Bypass Grafts, C. R. Bard, Murray Hill, NJ), which is fabricated from a porous polymer. FIG. 4 shows another example of a vascular graft 24 having a bifurcation 26.

In one variation, the drug-releasing vascular graft is manufactured by shape-forming the graft from a continuous porous polymeric tubing. Once the structure of the graft has taken shape, the vascular graft is immersed into an organic solvent to load the drug into the porous polymer, which forms the structure of the graft. At the end of the incubation period, the vascular graft is removed and the organic solvent is evaporated to created crystals in the porous polymer.

In another variation, the drug is loaded into a porous polymeric material prepared for fabricating a drug-releasing graft. For example, an ePTFE tubing can be immersed in an organic solution with a dissolved drug. The ePTFE tubing is then incubated for a period of time, after which the organic solvents are extracted therefrom. Organic solvents that remain on the ePTFE tubing are evaporated and crystals of the drug are formed in the ePTFE polymer. The ePTFE tubing embedded with drug crystals is then utilized to manufacture the vascular graft. For example, rings, spirals and expanded ends are heat-set onto the ePTFE tubing embedded with drug crystals. Further machining may also be implemented to modify the tubing to form the drug-releasing vascular graft.

### Controlled Release Study

With respect to a vascular cuff graft, examples of which are shown in FIGS. 2 and 3, experimentation was conducted to study the release of a drug without a polymeric carrier from the cuff surface. The drug chosen for the experiment was chlorhexidine acetate (ChAc) and the release time was scheduled for nine weeks. The materials and methods of the experiment included creating a calibration curve, preparing the vascular cuff grafts, and measuring absorbance, each of which is described in detail below.

Initially, a calibration curve was created to determine the concentration of ChAc in phosphate buffered saline (PBS). This curve was used to determine the concentration of the drug eluting from the vascular graft cuff pores. A stock solution of 0.01% weight/volume (w/v) was prepared and diluted to 0.0025%, 0.005%, and 0.0075% w/v. The absorbance of each of these solutions was determined using the Shimadzu UV-1601 spectrophotometer. A curve of absorbance versus concentration was expected to be linear. The data was fitted with a linear trend line with an expected R² value greater than 0.95. Table 1 lists the absorbance readings for the different concentrations of ChAc and Chart 1 shows the calibration curve with linear trend fit.

**Table 1**

| Concentration | Absorbance |
|---|---|
| 0.0025% | 0.101 |
| 0.005% | 0.227 |
| 0.0075% | 0.327 |
| 0.001% | 0.513 |
| 0.000% | 0.000 |

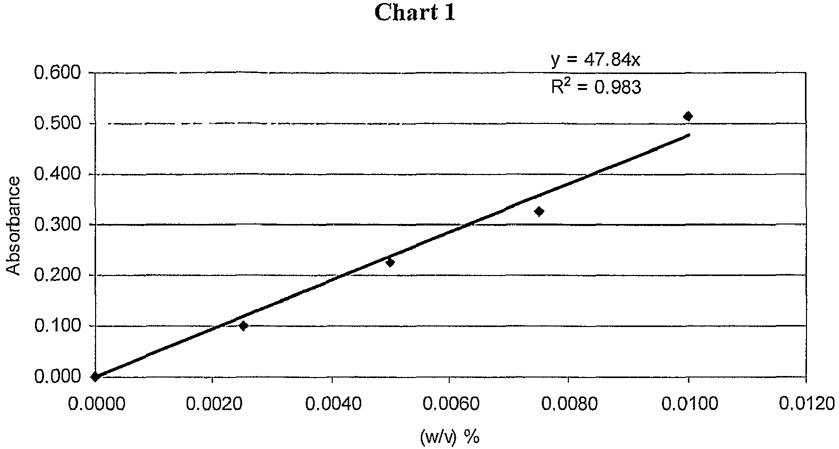

To prepare the vascular cuff grafts, the drug was embedded therein using the following procedure. First, 0.400 g ChAc was dissolved in 2 mL ethanol in a glass sample vial to make a 20% w/v solution. The sample was then vortexed and heated on a hot plate for a few minutes to completely dissolve the sample and the solution was transferred to a 50 mL centrifuge tube. Four 1 cm² sections from four vascular cuffs were cut with a razor blade. Three of the sections (1-3) were placed in the 20% solution and the remaining section (C) was utilized as an untreated control. The three cuff sections were soaked for two hours, removed, and allowed to dry overnight in a chemical hood. The cuff section weights with the loaded drug were then determined and recorded. The cuff section weights without the loaded drug were determined after the study by washing the cuffs with ethanol and drying. It is noted that the samples were not agitated while they were incubated at 37° C.

Absorbance was measured to determine the amount of drug released from the cuff sections. This was accomplished by utilizing two treated cuff section samples (1-2) and the one untreated cuff section sample (C). The remaining treated cuff section sample (3) was used for imaging by light microscopy and scanning electron microscopy (SEM). The untreated sample and two treated samples were placed in three 50 mL centrifuge tubes containing 5 mL PBS. Each centrifuge tube was labeled with the start time and time of removal. At different time points, cuff section samples were removed and placed in new PBS solutions. Different forceps were used to retrieve the control and treated samples in order to prevent contamination of the control sample. The PBS solutions were then analyzed using the Shimadzu UV 1600 spectrophotometer. Two milliliters of the solutions removed were used for measurements in the spectrophotometer. A reference cuvette with 2 mL of PBS was placed in the reference holder. Before each measurement, baseline correction was performed. The absorbance at ∼286 nm was recorded by automatic peak detection by the spectrophotometer. Concentration of drug was determined using the calibration curve (y = 47.84x) where y is the absorbance reading.

Table 2 provides an estimate of the amount of drug loaded into the samples, the data for which was collected after the nine week period. Table 3 lists the time points at which the samples were removed and placed in new PBS solutions. Table 4 summarizes all absorbance readings from the study. Chart 2 shows the percentage of drug released during the nine week period.

**Table 2**

| Sample | Weight (g) with drug | Weight (g) without drug | Drug loaded (mg) | Drug released - total (mg) |
|---|---|---|---|---|
| C | 0.0417 | 0.0407 | -- | -- |
| 1 | 0.0448 | 0.0370 | 7.8 | 7.399 |
| 2 | 0.0420 | 0.0342 | 7.8 | 6.388 |
| 3 | 0.0528 | -- | -- | -- |

**Table 3**

| | Date | Time | Time Elapsed |
|---|---|---|---|
| 1 | 9/13/05 | 8:45 AM | 30 min |
| 2 | 9/13/05 | 9:15 AM | 1 hour |
| 3 | 9/13/05 | 10:15 AM | 2 hours |
| 4 | 9/13/05 | 12:15 PM | 4 hours |
| 5 | 9/13/05 | 5:00 PM | 9 hours |
| 6 | 9/14/05 | 8:38 AM | 24 hours |
| 7 | 9/20/05 | 8:00 AM | 1 week |
| 8 | 9/27/05 | 8:00 AM | 2 weeks |
| 9 | 10/4/2005 | 8:00 AM | 3 weeks |
| 10 | 10/11/2005 | 8:00 AM | 4 weeks |
| 11 | 10/18/2005 | 8:00 AM | 5 weeks |
| 12 | 10/25/2005 | 8:00 AM | 6 weeks |
| 13 | 11/8/2005 | 9:00 AM | 8 weeks |
| 14 | 11/15/2005 | 8:00 AM | 9 weeks |

**Table 4**

| | Control | | Sample 1 | | Sample 2 | |
|---|---|---|---|---|---|---|
| | Absorbance | Wavelength (nm) | Absorbance | Wavelength (nm) | Absorbance | Wavelength (nm) |
| 1 | 0.000 | --- | 1.194 | 287.200 | 1.080 | 287.500 |
| 2 | 0.000 | --- | 0.630 | 285.800 | 0.352 | 286.900 |
| 3 | 0.009 | 285.000 | 0.335 | 286.500 | 0.275 | 286.500 |
| 4 | 0.009 | 287.400 | 0.500 | 286.800 | 0.501 | 287.400 |
| 5 | 0.099 | 286.000 | 0.614 | 287.300 | 0.574 | 287.600 |
| 6 | 0.000 | 287.000 | 0.707 | 287.800 | 0.688 | 287.400 |
| 7 | 0.003 | 288.500 | 0.638 | 287.500 | 0.605 | 287.300 |
| 8 | 0.013 | 287.700 | 0.621 | 287.300 | 0.579 | 287.200 |
| 9 | 0.014 | 287.000 | 0.578 | 287.100 | 0.517 | 287.000 |
| 10 | 0.007 | 287.000 | 0.462 | 286.800 | 0.436 | 287.000 |
| 11 | 0.004 | 287.000 | 0.357 | 286.800 | 0.295 | 286.700 |
| 12 | 0.000 | 287.000 | 0.213 | 286.800 | 0.080 | 286.900 |
| 13 | 0.031 | 287.200 | 0.100 | 286.400 | 0.072 | 285.500 |
| 14 | 0.032 | 287.200 | 0.130 | 286.700 | 0.058 | 285.900 |

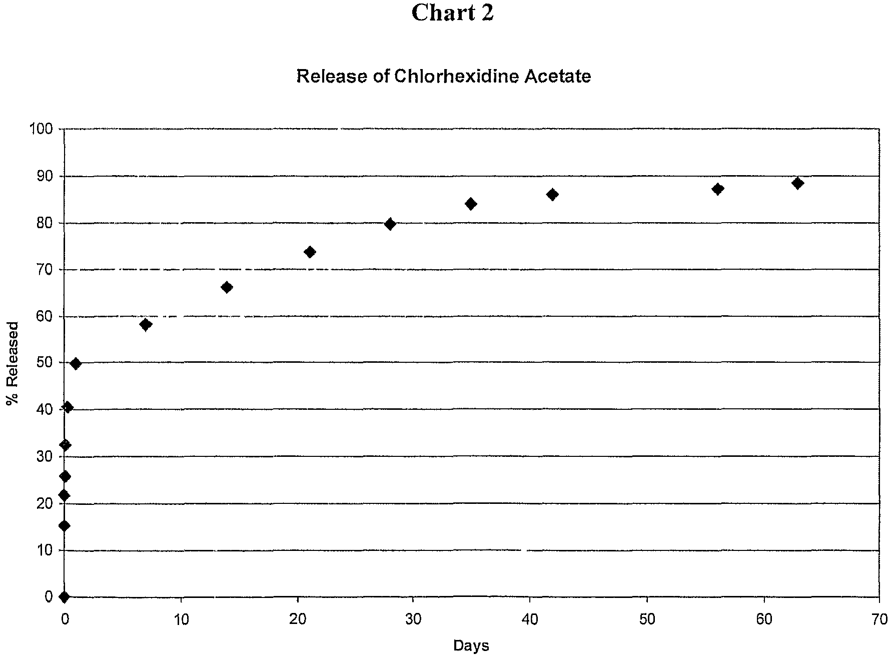

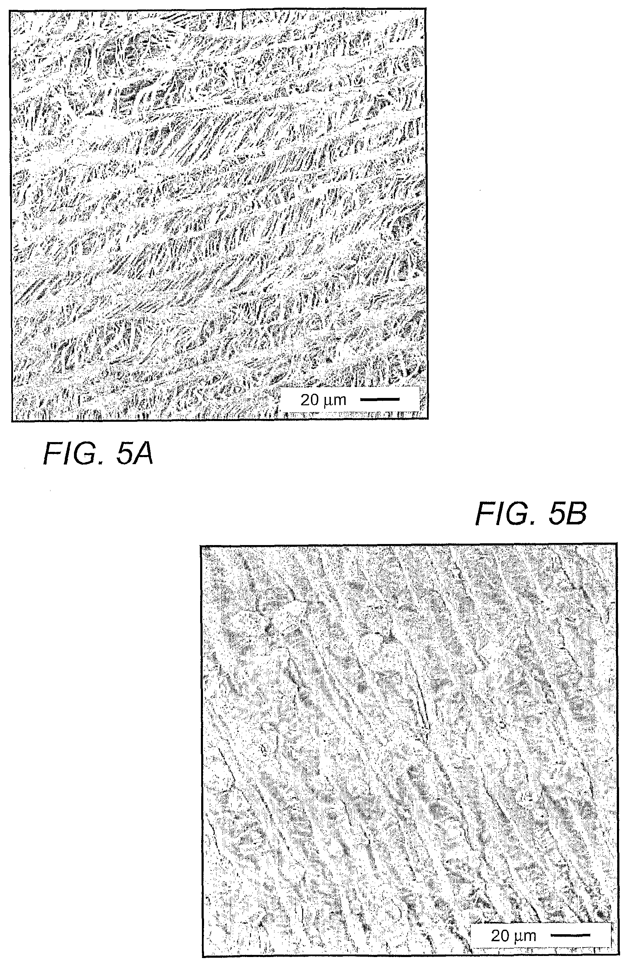

It was observed that the control sample cuff was highly hydrophobic, such that when placed in the PBS solution, the graft floated on the surface; however, the treated sample cuffs were less hydrophobic (more hydrophilic) and stayed in the solution, albeit near the surface. With respect to the ChAc, in the first time points some dissolution of the drug could be seen visually, and over the course of the study the ChAc slowly dissolved from the ePTFE cuff section. FIG. 5A is an SEM picture of the untreated control sample without any drug, showing the node-fibril microstructure of the ePTFE. FIG. 5B is an SEM picture of a treated sample with ChAc incorporated therein, showing large crystals in the microstructure of the ePTFE. The porosity of the ePTFE material provides a favorable environment for deposition of the drug crystals. By eliminating the use of a polymeric controlled release carrier for the drug, it is believed that the hemocompatibility of the graft surface is enhanced. The water insoluble drug ChAc was suitable for the experiment due at least in part because it is soluble in an organic solvent such as ethanol. Other water insoluble drugs, such as those mentioned herein, would also be suitable for incorporation into an ePTFE graft, as discussed above.

To summarize, a section of a cuff from an ePTFE vascular cuff graft was soaked in a solution of ChAc and the solution penetrated the porous microstructure of the material. The solvent was then evaporated, leaving behind water insoluble drug crystals in the material matrix. When incubated in an aqueous environment, the drug slowly dissolved. By embedding a water insoluble drug in a vascular graft, sustained delivery of the drug can be achieved over long periods of time. In this experiment, although approximately 50% of the drug was released after one day, it is believed to be attributed to the loss of drug particles on the outer surface of the graft that were easily shed. After nine weeks, levels of the drug were still detectable indicating sustained delivery. The release rate of the drug appears to be linear before leveling off as the concentration decreased.

In another embodiment, a drug-releasing graft 28 is configured with a tubular shaped porous polymeric structure 30 with embedded flexible metal alloy 32 (e.g., nitinol, etc.), as shown in FIG. 6. In one variation, the drug crystals are embedded into the polymer portion of the graft before the lattice is incorporated with the polymer to form the graft. In another variation, the complete polymer-lattice structure of the graft is fabricated first before the drug crystals are form within the polymeric portion of the graft through method described above. FIG. 7 illustrates yet another embodiment of a drug-releasing graft. In this embodiment, the drug-releasing graft includes a stent graft 34 having an expandable lattice 36 covered by a porous polymer sheet 38. The drug crystals are loaded within the pores of the porous polymeric sheet.

Examples of methods for fabricating vascular grafts with porous polymeric materials are disclosed in PCT Application, Publication No. WO 00/71179 A1, titled "EXPANDED POLYTETRAFLUOROETHYLENE VASCULAR GRAFT WITH INCREASED HEALING RESPONSE" by Edwin, et al., published Nov. 30, 2000; U.S. Patent Application Publication No. 2004/0037986 A1, titled "BLOOD-FLOW TUBING" by Houston et al., published Feb. 26, 2004; U.S. Patent No. 5,749,880, titled "ENDOLUMINAL ENCASULATED STENT AND METHODS OF MANUFACTURE AND ENDOLUMINAL DELIVERY" issued to Banas et al., dated May 12, 1998; U.S. Patent No. 6,245,099 B1, titled "SELECTIVE ADHERENCE OF STENT-GRAFT CONVERINGS, MANDREL AND METHOD OF MAKING STENT-GRAFT DEVICE" issued to Edwin et al., dated Jun. 12, 2001.

In view of the disclosure herein, one skilled in the art would appreciate that a drug-releasing graft can be fabricated with various suitable porous polymeric materials. Examples of porous polymeric materials that can be utilized to fabricate a drug-releasing graft includes, but not limited to, porous polytetrafluoroethylene, porous high-density polyethylene, porous polyurethane, copolymers of poly (2-hydroxyethyl methacrylate), and other biocompatible micro-porous polymers. In addition, porous biodegradable polymer can also be utilized in the manufacturing of the drug-releasing graft. For example, the method described above can be used to form drug crystals in a medical device comprising porous poly-lactic acid (PLA), poly-glycolic acid (PGA), poly-lactic-co-glycolic acid (PLGA), or a combination of polymers thereof. One skilled in the art, having the benefit of this disclosure would also appreciate that the methods described herein can be utilized to form drug crystals in a mesh-like material in a medical device. Alternatively, drug crystals can be formed in a mesh-like material that is later utilized to fabricate a medical device for implantation.

While the invention has been described in terms of particular variations and illustrative figures, those skilled in the art will recognize that the invention is not limited to the variations or figures described herein. In addition, where methods and steps described above indicate certain events occurring in certain order, those skilled in the art will recognize that the ordering of certain steps may be modified and that such modifications are in accordance with the variations of the invention. Additionally, certain of the steps may be performed concurrently in a parallel process when possible, as well as performed sequentially as described above. Therefore, to the extent there are variations of the invention, which are within the scope of the claims, it is the intent that this patent will cover those variations as well. Finally, all publications and patent applications cited in this specification are referred to.

## Claims

1. A method of loading a drug into an implantable medical device (20) which is a vascular graft of ePTFE having a node and fibril porous structure, the method comprising:
incorporating a water insoluble drug in a structure of the implantable medical device; and
forming crystals of the water insoluble drug in the structure of the implantable medical device, in interstices between the nodes and the fibrils of the structure of the ePTFE graft.

2. The method according to claim 1, wherein the step of incorporating a water insoluble drug comprises:
providing an organic solvent with the water insoluble drug dissolved therein;
placing the implantable medical device into the organic solvent; and
incubating the implantable medical device in the organic solvent for a period of time.

3. The method according to claim 2, wherein the step of incorporating a water insoluble drug further comprises allowing the organic solvent to evaporate from the implantable medical device.

4. The method according to claim 1, wherein the water insoluble drug comprises a cell cycle inhibitor.

5. The method according to claim 2 or 3, wherein the incubating step further comprises facilitating the organic solvent to permeate at least a portion of the medical device by inducing a pressure gradient across the body of the implantable medical device.

6. The method according to claim 1, wherein the water insoluble drug comprises a substance that reduces smooth muscle proliferation.

7. The method according to claim 1, wherein the water insoluble drug comprises an anti-thrombosis agent.

8. The method according to claim 2, claim 3 or claim 5, wherein the period of time of incubating the implantable medical device is five minutes or more.

9. An implantable medical device (20) which is a vascular graft of ePTFE having a node and fibril porous structure, the device comprising:
an elongated generally tubular structure (30) comprising the ePTFE polymer; and
a plurality of crystals embedded within a wall of the ePTFE polymer, in interstices between the nodes and the fibrils, with each of the plurality of crystals comprising a water insoluble drug.

10. The implantable medical device according to claim 9, wherein the water insoluble drug comprises a substance that suppresses smooth muscle growth.

11. The implantable medical device according to claim 10, wherein the substance comprises paclitaxel, or another cellular growth inhibitor.

12. The implantable medical device according to claim 9, 10 or 11, wherein the water insoluble drug comprises an anti-thrombosis agent.

13. The implantable medical device according to any one of claims 9 to 12, wherein the generally tubular structure further comprises a lattice (32) of a metal alloy.

## Patentansprüche

1. Verfahren zum Laden eines Wirkstoffs in eine implantierbare medizinische Einrichtung (20), die einen Gefäßgraft aus ePTFE mit einer porösen Knoten- und Fibrillenstruktur aufweist, wobei das Verfahren umfasst:
Eingliedern eines wasserunlöslichen Wirkstoffs in eine Struktur der implantierbaren medizinischen Einrichtung; und
Ausbilden von Kristallen des wasserunlöslichen Wirkstoffs in der Struktur der implantierbaren medizinischen Einrichtung in Zwischenräumen zwischen den Knoten und den Fibrillen der Struktur des ePTFE-Grafts.

2. Verfahren nach Anspruch 1, bei dem der Schritt des Eingliederns eines wasserunlöslichen Wirkstoffs umfasst:
Bereitstellen eines organischen Lösungsmittels mit dem darin gelösten wasserunlöslichen Wirkstoff;
Platzieren der implantierbaren medizinischen Einrichtung in dem organischen Lösungsmittel; und
Inkubieren der implantierbaren medizinischen Einrichtung in dem organischen Lösungsmittel über einen Zeitraum.

3. Verfahren nach Anspruch 2, bei dem der Schritt des Eingliederns eines wasserunlöslichen Wirkstoffs ferner das Zulassen eines Verdampfens des organischen Lösungsmittels aus der implantierbaren medizinischen Einrichtung umfasst.

4. Verfahren nach Anspruch 1, bei dem der wasserunlösliche Wirkstoff einen Zellzyklus-Inhibitor aufweist.

5. Verfahren nach Anspruch 2 oder 3, bei dem der Inkubationsschritt ferner ein Durchdringen des organischen Lösungsmittels durch zumindest einen Abschnitt der medizinischen Einrichtung mittels eines Hervorrufens eines Druckgradienten über den Körper der implantierbaren medizinischen Einrichtung ermöglicht.

6. Verfahren nach Anspruch 1, bei dem der wasserunlösliche Wirkstoff eine Substanz aufweist, die eine Proliferation von glatten Muskelzellen vermindert.

7. Verfahren nach Anspruch 1, bei dem der wasserunlösliche Wirkstoff ein Anti-Thrombosemittel aufweist.

8. Verfahren nach Anspruch 2, 3 oder 5, bei dem der Zeitraum des Inkubierens der implantierbaren medizinischen Einrichtung 5 Minuten oder mehr ist.

9. Implantierbare medizinische Einrichtung (20), die einen Gefäßgraft aus ePTFE mit einer porösen Knoten- und Fibrillenstruktur ist, wobei die Einrichtung aufweist:
eine längliche im Allgemeinen rohrförmige Struktur (30) die das ePTFE-Polymer aufweist; und
eine Vielzahl von Kristallen, die in einer Wand des ePTFE-Polymers in Zwischenräumen zwischen den Knoten und den Fibrillen eingebettet ist, wobei jeder der Vielzahl von Kristallen einen wasserunlöslichen Wirkstoff aufweist.

10. Implantierbare medizinische Einrichtung nach Anspruch 9, bei welcher der wasserunlösliche Wirkstoff eine Substanz aufweist, welche das Wachstum glatter Muskulatur unterdrückt.

11. Implantierbare medizinische Einrichtung nach Anspruch 10, bei der die Substanz Paclitaxel oder einen anderen zellulären Wachstumsinhibitor aufweist.

12. Implantierbare medizinische Einrichtung nach Anspruch 9, 10 oder 11, bei welcher der wasserunlösliche Wirkstoff ein Anti-Thrombosemittel aufweist.

13. Implantierbare medizinische Einrichtung nach einem der Ansprüche 9 bis 12, bei der die im Allgemeinen rohrförmige Struktur ferner ein Gitter (32) aus einer Metalllegierung aufweist.

## Revendications

1. Procédé de chargement d'un médicament dans un dispositif médical implantable (20) qui est un greffon vasculaire d'ePTFE comportant un noeud et une structure fibrillaire poreuse, le procédé comprenant :
l'incorporation d'un médicament insoluble dans l'eau à une structure du dispositif médical implantable ; et
la formation de cristaux du médicament insoluble dans l'eau dans la structure du dispositif médical implantable, dans des interstices entre les noeuds et les fibrilles de la structure du greffon d'ePTFE.

2. Procédé selon la revendication 1, dans lequel l'étape d'incorporation d'un médicament insoluble dans l'eau comprend :
la fourniture d'un solvant organique avec le médicament insoluble dans l'eau qui y est dissous ;
la mise en place du dispositif médical implantable dans le solvant organique ; et
l'incubation du dispositif médical implantable dans le solvant organique pendant une certaine période de temps.

3. Procédé selon la revendication 2, dans lequel l'étape d'incorporation d'un médicament insoluble dans l'eau comprend en outre le fait de laisser le solvant organique s'évaporer du dispositif médical implantable.

4. Procédé selon la revendication 1, dans lequel le médicament insoluble dans l'eau comprend un inhibiteur de cycle cellulaire.

5. Procédé selon la revendication 2 ou 3, dans lequel l'étape d'incubation comprend en outre le fait de faciliter la perméation par le solvant organique d'au moins une partie du dispositif médical en induisant un gradient de pression en travers du corps du dispositif médical implantable.

6. Procédé selon la revendication 1, dans lequel le médicament insoluble dans l'eau comprend une substance qui réduit la prolifération des muscles lisses.

7. Procédé selon la revendication 1, dans lequel le médicament insoluble dans l'eau comprend un agent anti-thrombotique.

8. Procédé selon la revendication 2, la revendication 3 ou la revendication 5, dans lequel la période de temps d'incubation du dispositif médical implantable est de cinq minutes ou plus.

9. Dispositif médical implantable (20) qui est un greffon vasculaire d'ePTFE comportant un noeud et une structure fibrillaire poreuse, le dispositif comprenant :
une structure tubulaire généralement allongée (30) comprenant le polymère d'ePTFE ; et
une pluralité de cristaux noyés dans une paroi du polymère de PTFEe, dans des interstices entre les nceuds et les fibrilles, chacun de la pluralité de cristaux comprenant un médicament insoluble dans l'eau.

10. Dispositif médical implantable selon la revendication 9, dans lequel le médicament insoluble dans l'eau comprend une substance qui supprime la croissance des muscles lisses.

11. Dispositif médical implantable selon la revendication 10, dans lequel la substance comprend du paclitaxel ou un autre inhibiteur de croissance cellulaire.

12. Dispositif médical implantable selon la revendication 9, 10 ou 11, dans lequel le médicament insoluble dans l'eau comprend un agent anti-thrombotique.

13. Dispositif médical implantable selon l'une quelconque des revendications 9 à 12, dans lequel la structure généralement tubulaire comprend en outre un treillis (32) d'un alliage métallique.
